# EUROPÄISCHE PATENTANMELDUNG

(11) **EP 2 397 169 A1**
(43) Veröffentlichungstag der Anmeldung: **21.12.2011**
(21) Anmeldenummer: 10165924.1
(22) Anmeldetag: 15.06.2010
(51) Int. Cl.: A61M 5/00

(54) **Halterung für eine Injektionsspritze**

(71) Anmelder: CSL Behring GmbH, 35041 Marburg (DE)
(72) Erfinder: Plünnecke, Dieter, 34233 Fuldatal (DE)
(74) Vertreter: Binsack, Beate

(57) **Zusammenfassung**

Die vorliegende Erfindung betrifft eine Halterung für eine Injektionsspritze (18) mit einem zur Aufnahme der Spritze ausgebildeten Gehäuse (12), einem Standfuß (14) zum Abstellen auf einer Unterlage und mit Haltemitteln (38, 52, 54, 62, 64) zum axialen und/oder radialen Fixieren der Spritze (18) an oder in der Halterung, wobei die Haltemittel (38, 52, 54, 62, 64) derart ausgebildet sind, dass ein Auslass (26) einer in der Halterung angeordneten Spritze (18) dem freien Ende des Standfußes (14) zugewandt und beabstandet hiervon zu liegen kommt.

## Beschreibung

Die vorliegende Erfindung betrifft eine Halterung für eine Spritze zu Selbstmedikationszwecken, insbesondere zum Abstellen auf einer Unterlage.

Insbesondere im Rahmen einer intravenösen oder subkutanen Darreichungsform von Medikamenten
wird der zumeist flüssige Wirkstoff mit Hilfe einer Spritze oder vergleichbaren Injektionsgeräten dem Körpergewebe zugeführt. Insbesondere bei der Selbstmedikation oder Heimtherapie erfordern derartige Medikamente eine entsprechende Schulung des Patienten. Soll beispielsweise ein Hämophilie-Präparat verabreicht werden, so geschieht dies zumeist unter Verwendung einer sogenannten Butterfly-Kanüle.

Diese legt sich ein Patient selbst z.B. im Bereich der Armbeuge. Für den eigentlichen Injektionsvorgang steht dem Patienten dann jedoch nur noch eine Hand zur Verfügung. Hinzu kommt, dass das zu verabreichende Präparat eine im Vergleich zu Wasser höhere Viskosität aufweist, so dass der Patient zur Injektion des Wirkstoffs eine erhebliche Kraft auf einen Spritzenkolben ausüben muss. Eine derartige einhändige Handhabung der Injektionsspritze ist vergleichsweise umständlich und beschwerlich. Dabei besteht insbesondere die Gefahr, dass ein den Spritzenauslass mit einer Injektionskanüle verbindender fluidführender Schlauch beim Injektionsvorgang abgeknickt wird, was die Zuführung des Präparats weiter erschwert.

Weiter gilt es zu berücksichtigen, dass insbesondere Hämophilie-Patienten bereits an Sekundärerkrankungen leiden und hinsichtlich ihrer Beweglichkeit und Fingerfertigkeit oftmals eingeschränkt sind. Versteifte Gelenke gerade im Unterarm- und Handbereich können die Selbstmedikation erheblich erschweren.

Es ist daher Aufgabe der vorliegenden Erfindung, eine Halterung für eine Injektionsspritze bereitzustellen, die eine einfache, sichere sowie zuverlässige Injektion ermöglicht und die Handhabung einer Injektionsspritze bzw. eines Injektionssets, umfassend einen Injektionsschlauch, eine Kanüle und eine Spritze vereinfacht.

Die der Erfindung zugrundeliegende Aufgabe wird mit Hilfe einer Halterung gemäß dem unabhängigen Patentanspruch 1 gelöst. Einzelne vorteilhafte Ausgestaltungen der Erfindung sind Gegenstand der abhängigen Patentansprüche.

Die erfindungsgemäße Halterung ist insbesondere für eine Injektionsspritze ausgebildet. Sie weist ein zur Aufnahme der Spritze ausgebildetes Gehäuse und einen Standfuß zum Abstellen der Halterung auf einer Unterlage auf. Ferner sind an der Halterung Haltemittel zum axialen und/oder radialen Fixieren der Spritze an oder in der Halterung vorgesehen. Die Haltemittel sind dabei derart ausgebildet, dass ein Auslass der Spritze in der Halterung dem freien Ende des Standfußes zugewandt und beabstandet vom Standfuß, das heißt entsprechend beabstandet von der Unterlage, auf welcher die Halterung abzustellen ist, zu liegen kommt. Dabei ist insbesondere vorgesehen, dass die Haltemittel der Halterung für eine Spritzenfixierung innerhalb oder an der Halterung ausgelegt sind, sodass der Spritzenauslass bei einer auf einer Unterlage abgestellten Halterung zur Unterlage hin orientiert, aber beabstandet hiervon angeordnet ist.

Durch die Möglichkeit, die Halterung quasi senkrecht auf einer Unterlage abzustellen, wird bereits eine vereinfachte Handhabung der Spritze zu Injektionszwecken bereitgestellt. So kann der Anwender beispielsweise die Halterung mit vier Fingern umgreifen, während er mit dem Daumen auf den ausgezogenen Spritzenstößel eine nach unten gerichtete Injektionskraft ausübt. Hierbei erweist es sich von Vorteil, dass die Spritzenhalterung auf einer Unterlage sicher abgestellt werden kann und die zu Injektionszwecken auf den Spritzenstößel auszuübenden Kräfte über den Spritzenkörper in die Halterung und schließlich auf oder in die Unterlage abgeleitet werden können. Sollte der Anwender etwa alleine mit seinem Daumen die erforderliche Injektionskraft nicht aufbringen können, so besteht mittels der Halterung ferner die Möglichkeit, mit anderen Körperpartien, etwa mit der flachen Hand, Druck auf den Spritzenstößel auszuüben, ohne dass eine erhöhte Gefahr bestünde, den die Kanüle und die Spritze fluidführend miteinander verbindenden Schlauch abzuknicken.

Der Standfuß ist von Vorteil zum Abstellen auf einer im Wesentlichen ebenen Unterlage, etwa einem Tisch, ausgebildet. Der Fuß kann hierzu beispielsweise einen zumindest bereichsweise umlaufenden Aufstellrand aufweisen, der im Wesentlichen in der Ebene senkrecht zur Spritzenlängsrichtung liegt.

So ist nach einer ersten bevorzugten Ausgestaltung der Erfindung vorgesehen, dass die für die Spritze vorgesehenen halterseitigen Haltemittel eine derartige axiale Fixierung der Spritze in oder an der Halterung ermöglichen, dass der Abstand zwischen Spritzenauslass zum Standfußende wenigstens der Axialerstreckung eines Schlauchanschlussstücks zuzüglich eines Mindest-Schlauchbiegeradius entspricht, bei welchem der mit dem Spritzenauslass in Fluidverbindung tretende Schlauch fluiddurchlässig bleibt. Auf diese Art und Weise kann erreicht werden, dass der am Spritzenauslass anzuordnende Schlauch quasi frei hängend in der die Spritze aufnehmenden Halterung liegt. Ein Abknicken des fluidführenden Schlauchs wird somit effektiv verhindert. Nach einer Weiterbildung der Erfindung ist ferner vorgesehen, dass das Gehäuse der Halterung an seinem dem Standfuß abgewandten oberen Endabschnitt einen radial nach außen ragenden, zumindest bereichsweise umlaufenden Anlageflansch aufweist. Mit diesem Anlageflansch kann ein Hindurchrutschen des Gehäuses durch eine das Gehäuse umfassende Hand des Anwenders effektiv verhindert werden. In Anwendungsszenarien, bei welchen mittels des Daumens eine Injektionskraft auf den Spritzenstößel aufzubringen ist, umschließen die übrigen vier Finger der Hand das Gehäuse der Halterung. Der radial nach außen ragende und zumindest bereichsweise umlaufende Anlageflansch kommt dabei bevorzugt an der dem Daumen zugewandten Seite eines Zeigefingers zu liegen.

Nach einer Weiterbildung der Erfindung ist ferner vorgesehen, dass das Gehäuse eine zumindest bereichsweise zylindrische, mit einer außenseitigen Grifffläche versehene Grundstruktur aufweist, die sich zum Standfuß hin radial erweitert. Die Grifffläche selbst kann beispielsweise an die Ergonomie einer menschlichen Hand angepasst sein. Sie kann beispielsweise eine dementsprechende Oberflächenstruktur, etwa eine an die einzelnen Finger einer menschlichen Hand angepasste Riffelung aufweisen. Abweichend davon kann sie natürlich auch eine im Wesentlichen glatte, bereichsweise kreisrunde oder ovale Kontur aufweisen.

Die radiale Erweiterung des Gehäuses verbessert die Standsicherheit des Gehäuses auf der Unterlage. So kann beispielsweise vorgesehen sein, dass sich das Gehäuse zum Standfuß hin konisch oder anderweitig radial erweitert. So kann selbst bei Aufwendung höchster Injektionskräfte ein unbeabsichtigtes Umfallen der Halterung oder ein Um- oder Abknicken des Spritzenschlauchs während eines Injektionsvorganges weitgehend verhindert bzw. ausgeschlossen werden.

In einer besonderen Ausführungsform werden das Gehäuse, der Standfuß und/oder der dem Standfuß gegenüberliegende Anlageflansch einstückig ausgebildet. Bevorzugt ist hier vorzusehen, das Gehäuse, den Standfuß und/oder den Anlageflansch in Form eines einzigen Spritzgussteils, bevorzugt als Kunststoff-Spritzgussbauteil auszubilden. Die Ausbildung als Kunststoff-Spritzgussbauteil ist vergleichsweise einfach und kostengünstig zu verwirklichen.

Weiterhin ist nach einer bevorzugten Ausführungsform der Erfindung vorgesehen, dass das Gehäuse eine radial zugängliche Aufnahme zum Einfügen der Spritze in das Gehäuse aufweist. Betrachtet man die Zylindersymmetrie der Spritze bzw. des das Medikament aufnehmenden Spritzenkörpers, so ist vorgesehen, die Spritze transversal zur Spritzenlängsrichtung, das heißt transversal zur Axialrichtung, in die Aufnahme des Gehäuses einzusetzen. Dementsprechend weist das Gehäuse keine strikte Zylindersymmetrie auf, sondern ist in Umfangsrichtung nur bereichsweise rund ausgebildet, während es zu der für die Spritze vorgesehenen Einführöffnung hin, beispielsweise als aufgeschnittener Hohlzylinder ausgebildet ist, der mittels eines gesonderten Verschlussmechanismus nach Einsetzen einer Spritze zum Fixieren derselben verschließbar ist.

Alternativ oder ergänzend hierzu ist ferner aber auch denkbar, die Spritze in Axialrichtung, das heißt in Spritzenlängsrichtung gesehen, in die Halterung von oben einzuführen, bis die Spritze mit ihren am oberen Ende des Spritzenkörpers nach außen auskragenden Haltevorsprüngen an dafür vorgesehenen Anschlagsflächen oder Auflagerflächen der Halterung zu liegen kommt. Die Spritze wäre dabei unter Umständen lediglich unidirektional in Axialrichtung, nämlich in Injektionsrichtung an der Halterung fixiert, während die Halterung selbst als hohlzylindrische Aufnahme für den Spritzenkörper fungiert.

Bevorzugt ist für die Erfindung vorgesehen, dass die Halterung unterschiedlich große Spritzen aufnehmen kann. Die Halterung soll insbesondere für 5-, 10-oder 20-Milliliter-Einwegspritzen ausgebildet sein und sämtliche, zumindest aber die drei genannten Spritzenformate, sowohl in Radial- als auch Axialrichtung zu Injektionszwecken fixieren können. Hierzu erweist es sich als vorteilhaft, wenn in der Spritzenaufnahme der Halterung zumindest ein Halteflügel schwenkbar gelagert ist, der mit dem Spritzenkörper mit Einsetzen der Spritze in die Aufnahme klemmend zur Anlage bringbar ist, d.h. mit welchem die Spritze klemmend in die Halterung bringbar ist. Der zumindest eine Halteflügel stellt eine Art radialen Rastmechanismus für die Spritze zur Verfügung und ermöglicht, dass sämtliche der vorgenannten Spritzengrößen mit jeweils unterschiedlichen Durchmessern in der Spritzenaufnahme der Halterung fixiert werden können.

Von Vorteil ist dabei vorgesehen, dass der zumindest eine Halteflügel an einer im Wesentlichen parallel zur Axialrichtung der Halterung, bzw. parallel zur Längserstreckung der Spritze verlaufenden Schwenkachse unter Einwirkung zumindest eines Federelements, vorzugsweise einer Drehfeder, gelagert ist. So kann vorgesehen sein, den Halteflügel beim Einsetzen der Spritze entgegen der Federkraft zu verschwenken, sodass mit Erreichen einer Endmontagestellung der Spritze in der Halterung der Halteflügel mit Hilfe der von der Feder aufgebrachten Haltekraft die Spritze zumindest in Axialrichtung in der vorgegebenen Position hält.

Nach einer bevorzugten Ausgestaltung sind zwei parallel zueinander verlaufende, schwenkbar an der Halterung angelenkte Halteflügel vorgesehen, die je nach Schwenkstellung eine Spritzenaufnahme variabler Größe bilden. Durch das Vorsehen zweier Halteflügel können unterschiedlich große Spritzenkörper in der Halterung problemlos fixiert werden. Es erweist sich dabei ferner von Vorteil, wenn die Halteflügel in der Ebene senkrecht zur Axialrichtung, das heißt senkrecht zur Ebene ihrer Schwenkachse zumindest bereichsweise eine dem Spritzenkörper angepasste Krümmung aufweisen. Die Krümmung der Halteflügel ist von innen, von der Aufnahme her betrachtet, bevorzugt konvex, sodass der in Umfangsrichtung typischerweise rund ausgebildete Spritzenkörper möglichst großflächig an den Halteflügeln zur Anlage gelangt. Je nach bevorzugtem Spritzentyp ist die Krümmung der Halteflügel an die Krümmung des Spritzenkörpers anzupassen oder sie ist hieran angepasst.

Nach einer weiteren bevorzugten Ausgestaltung der Erfindung ist ferner vorgesehen, dass der zumindest eine Halteflügel an einem seiner Schwenkachse entgegengesetzten, also an seinem freien Endabschnitt einen im Wesentlichen radial nach außen ragenden und als Einführhilfe für die Spritze fungierenden Endabschnitt aufweist. In Montagerichtung der Spritze gesehen verjüngt sich der Halteflügel nach innen, bevor er eine konkav gekrümmte Aufnahme für den Spritzenkörper bildet.

Beim Einführen der Spritze kann der zum freien Ende der Halteflügel sich radial erweiternde Endabschnitt nicht nur als Einführhilfe dienen, sondern etwa beim Einsetzen des Spritzenkörpers in die Aufnahme dafür Sorge tragen, dass die Halteflügel nach außen verschwenken und somit erst die Aufnahme für den Spritzenkörper freigeben. Nachdem der Spritzenkörper mit seinem vollen Radius jene Taillierung passiert hat, können die Halteflügel unter Einwirkung der von der Drehfeder bereitgestellten Haltekraft wieder in Richtung ihrer ursprünglichen Konfiguration verschwenken und den Spritzenkörper zwischen sich einklemmen.

Nach einer weiteren vorteilhaften Ausgestaltung der Erfindung ist ferner vorgesehen, dass die für die Spritze vorgesehene Aufnahme mittels eines schwenkbar oder drehbar am Gehäuse gelagerten Verschlussmittels verschließbar ist. Eine derartige Konfiguration ist insbesondere dann vorgesehen, wenn das Gehäuse der Halterung nur bereichsweise zylindrisch, nach einer Seite hin offen, zum radialen Einsetzen der Spritze ausgebildet ist.

In einer bevorzugten Ausführungsform ist das Verschlussmittel dabei als klappenartiger Verschluss ausgebildet, der an einer Seite der Spritzenaufnahme schwenkbar angelenkt und nach Einsetzen der Spritze in die Aufnahme zur gegenüberliegenden Wange des Gehäuses schwenkbar und dort fixierbar ist.

Als Fixierung ist hierbei vorzugsweise ein Rastmechanismus von Gehäusewange und verschwenkbarem Verschlussmittel vorzusehen. Das Verschlussmittel selbst ist dabei bevorzugt als ein die Spritzenaufnahme in Umfangsrichtung des Gehäuses und in Spritzenlängsrichtung zumindest bereichsweise umschließendes Rastmittel ausgebildet. Dieses wirkt zum Verschließen der Spritzenaufnahme mit einem außen am Gehäuse bzw. an der entsprechenden Gehäusewange angeordneten Gegenrastmittel zusammen. Wenngleich die Spritze bereits in Radialrichtung gesehen mit Hilfe des zumindest einen Halteflügels an der Halterung fixiert werden kann, so bildet das schwenkbar am Gehäuse angeordnete Verschlussmittel eine weitere Sicherung gegen unbeabsichtigtes Lösen der Spritze aus dem Gehäuse bzw. von der Halterung.

Nach einer weiteren vorteilhaften Ausgestaltung der Erfindung ist ferner vorgesehen, dass das schwenkbar am Gehäuse gelagerte Verschlussmittel an seiner der Spritze zugewandten Innenseite zumindest eine Zahnfläche oder Verzahnung aufweist, welche dazu ausgebildet ist, mit einer korrespondierend hierzu ausgebildeten endseitigen Schrägfläche oder Fase des zumindest einen Halteflügels derart zusammenzuwirken, dass der Halteflügel unter Einwirkung einer radial nach innen gerichteten Kraft auf das Verschlussmittel radial nach innen zur Spritze hin verschwenkt. Mit anderen Worten wirkt das Verschlussmittel innenseitig mit dem radial nach außen gerichteten freien Endabschnitt zumindest eines, bevorzugt zweier Halteflügel zusammen, wobei durch eine nach innen gerichtete Druckausübung auf das Verschlussmittel der Halteflügel radial nach innen verschwenkt und so eine Klemmwirkung zwischen Halteflügel und Spritze herbeigeführt werden kann.

Es erweist sich hierbei als besonders vorteilhaft, wenn das Verschlussmittel zwei in Umfangsrichtung nebeneinander angeordnete und flexibel miteinander verbundene Flächensegmente aufweist, welche jeweils mit einer innenliegenden Zahnfläche mit einer endseitigen Schrägfläche eines Halteflügels zusammenwirken. Die beiden Flächensegmente des Verschlussmittels sind in Umfangsrichtung gesehen nebeneinanderliegend angeordnet und können entlang einer in Axialrichtung verlaufenden Achse gegeneinander verschwenken. Mittels dieser Schwenkbarkeit oder Knickbarkeit des Verschlussmittels kann jedes der nach innen, zumindest bereichsweise verzahnten Flächensegmente eine radial nach innen gerichtete Kraft auf die schwenkbar in der Aufnahme angeordneten Halteflügel ausüben. Auf diese Art und Weise wird ferner ermöglicht, dass ein und dieselbe Halterung zur Fixierung unterschiedlich großer Spritzen fungieren kann.

In einer alternativ bevorzugten Ausführungsform ist das Verschlussmittel als ein um die Axialrichtung im Vergleich zum Gehäuse drehbarer Verschluss ausgebildet, wobei im Verschlussmittel ein Bereich ausgebildet ist, der nach partieller Drehung um die Spritzenlängsrichtung die Spritze in der Aufnahme des Gehäuses fixiert. Der drehbare Verschluss kann dabei beispielsweise als eine Art offener Ring ausgebildet sein, der die Spritzenaufnahme zumindest teilweise radial und mit einer Öffnung umschließt und der nach Einsetzen der Spritze in die Aufnahme um die Spritzenlängsrichtung drehbar ist, wodurch die Öffnung des Ringes seitlich verdreht und dadurch die Spritze in der Aufnahme fixiert wird. Die Handhabung, insbesondere die einhändige Handhabung, des drehbaren Verschlussmittels erweist sich als besonders einfach und daher besonders vorteilhaft.

Bei weiteren bevorzugten Ausgestaltungen der Halterung ist ferner vorgesehen, dass das Verschlussmittel den Bereich der Spritzenaufnahme in Axialrichtung gesehen nur bereichsweise überdeckt. Insbesondere soll der unten, dem Standfuß zugewandte Spritzenabschnitt nicht vom Verschlussmittel überdeckt werden, sondern sichtbar bleiben, damit der Anwender den Füllstand der Spritze während des Injektionsvorgangs visuell überprüfen kann.

Des Weiteren ist für die Erfindung ferner vorgesehen, dass der zumindest eine Halteflügel und/oder das Gehäuse selbst an seinem oberen, dem Standfuß abgewandten Endabschnitt zumindest einen Schlitz oder ein Auflager für einen radial nach außen ragenden Haltevorsprung bzw. für zwei diametral in Radialrichtung auskragende Haltevorsprünge einer Spritze aufweist, um die Spritze zumindest in Injektionsrichtung betrachtet, axial an der Halterung zu fixieren.

Für den im oberen Abschnitt der Halteflügel vorgesehenen Schlitz kann ferner eine Einführschräge für die Spritzen-Haltevorsprünge vorgesehen werden, sodass mit Einsetzen der Spritze in die Aufnahme auch im Bereich der Haltevorsprünge und den halteflügelseitigen Schlitzen bereits eine Klemmwirkung erzielt werden kann. Sofern in den Halteflügeln bereits Aufnahmeschlitze für die spritzenseitigen Haltevorsprünge vorgesehen sind, kann die Spritze bidirektional in Axialrichtung an der Halterung fixiert werden.

Bei Ausgestaltungen der Halterung ohne Halteflügel oder ohne Schlitze in den Halteflügeln, kann es aber auch ausreichen, wenn das zumindest bereichsweise umlaufend ausgebildete Gehäuse der Halterung zumindest etwas unterhalb des radial nach außen ragenden Anlageflanschs ein Auflager für die spritzenseitigen Haltevorsprünge bildet. Die Spritze wird somit beim Abstellen der Halterung auf einer Unterlage allein durch ihre Gewichtskraft und bei Ausübung einer auf den Spritzenkolben einwirkenden Injektionskraft jeweils mit ihren Haltevorsprüngen am gehäuseseitigen Auflager in Axialrichtung fixiert, etwa indem sie sich hieran abstützt.

Weitere Ziele, Merkmale sowie vorteilhafte Ausgestaltungen der Erfindung werden in der nachfolgenden Beschreibung von Ausführungsbeispielen unter Bezugnahme auf die Figuren erörtert. Dabei bilden sämtliche im Text beschriebenen als auch in den Figuren bildlich dargestellten Merkmale sowohl in Alleinstellung als auch in jeglicher sinnvollen Kombination untereinander den Gegenstand der vorliegenden Erfindung.

Es zeigen:
- Fig. 1: eine perspektivische Darstellung einer Spritzenhalterung in einer ersten Ausführungsform mit geschlossenem Verschluss,
- Fig. 2: die Spritzenhalterung gemäß Fig. 1 mit geöffnetem Verschluss,
- Fig. 3: die Halterung gemäß Fig. 2 ohne darin eingesetzte Spritze,
- Fig. 4: eine perspektivische Darstellung einer weiteren Ausgestaltung einer Halterung mit in Radialrichtung geschlitzten Halteflügeln,
- Fig. 5: die Halterung gemäß Fig. 4 mit eingesetzter Spritze,
- Fig. 6: eine vergrößerte Detailansicht der Spritzenhalterung gemäß Fig. 5,
- Fig. 7: eine perspektivische Darstellung eines Querschnitts durch die Halterung gemäß der Fig. 4 bis 6 in Höhe der am Verschluss vorgesehenen Verzahnung und
- Fig. 8: eine teiltransparente rückwärtige Ansicht der Halterung gemäß der Fig. 4 bis 7.

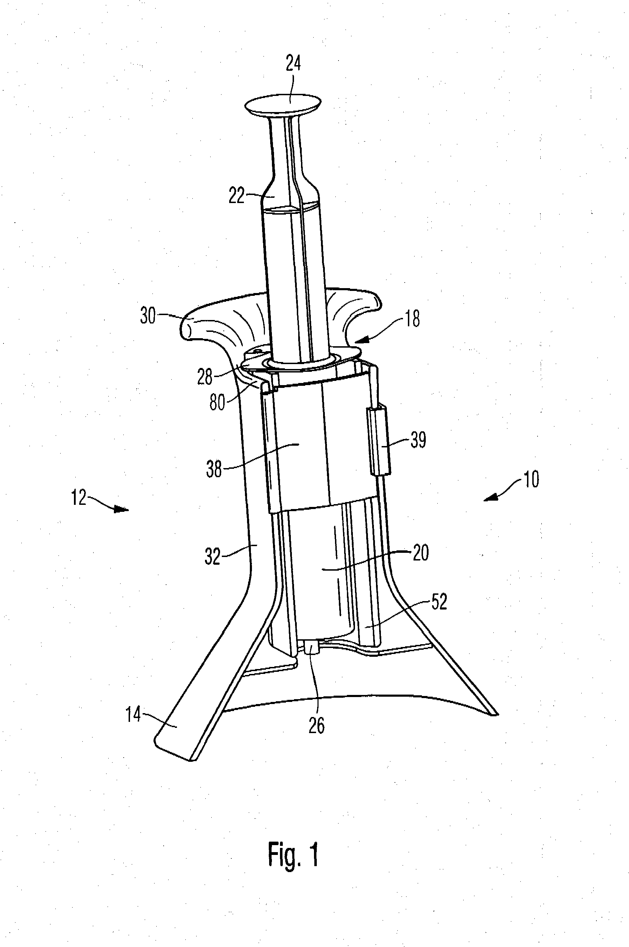

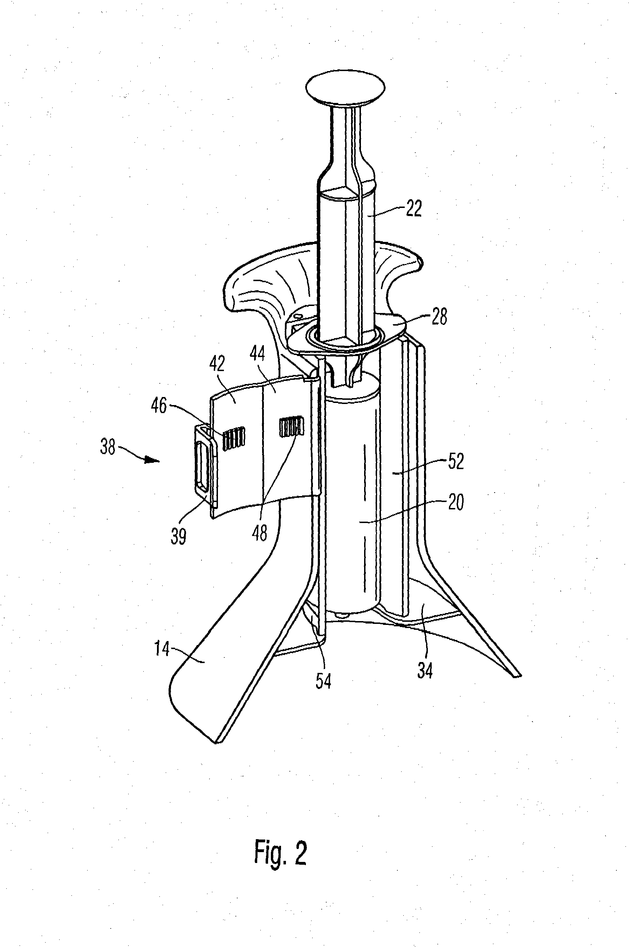

In den Fig. 1 bis 3 ist eine Halterung 10 zur Aufnahme einer Injektionsspritze 18 gezeigt. Die Halterung 10 weist eine Aufnahme 16, etwa für eine Einwegspritze 18 auf, die zum Beispiel einen im Wesentlichen zylindersymmetrischen Spritzenkörper 20 und einen darin axial verschiebbaren Spritzenkolben 22 aufnimmt. An der dem Spritzenauslass 26 des Spritzenkörpers 20 abgewandten Seite weist der Spritzenkolben 22 eine Druck-oder Kraftaufnahmefläche 24 auf, über welche beispielsweise der Anwender mit Hilfe seines Daumens die zum Injizieren einer Flüssigkeit benötigte Kraft auf den Spritzenkolben 22 ausüben kann.

Die Halterung selbst ist als Spritzgussbauteil, vorzugsweise aus thermoplastischem Kunststoff, ausgebildet. Die Halterung 10 weist ein Spritzengehäuse 12 auf, welches nach unten hin einen sich radial verbreiternden, etwa konisch verlaufenden Standfuß 14 und nach oben, dem Spritzenkolben 22 zugewandt, einen radial nach außen ragenden Anlageflansch 30 aufweist. Dazwischen weist das Gehäuse 12 eine Grifffläche 32 auf. Diese kann etwa der Innenkontur einer Handfläche ergonomisch angepasst sein. Zu Injektionszwecken ist von Vorteil vorgesehen, wenn der Anwender mit seinem Daumen von oben eine Kraft auf das Druckstück 24 ausübt und dabei mit der dem Daumen zugewandten Seite des Zeigefingers an der Unterseite des radial auskragenden Anlageflansches 30 abstützend zur Anlage gelangt.

Mittels des sich konisch oder radial erweiternden Abstell- oder Standfußes kann die gesamte Halterung 10 sicher auf einer, in den Figuren nicht explizit gezeigten Unterlage abgestellt werden. Dies erleichtert zum einen den Injektionsvorgang und ermöglicht es dem Anwender, die erforderliche Kraft für eine langsame und gleichmäßige Injektion kontrolliert aufzubringen. Wesentlich hierbei ist es, dass der Spritzenauslass 26 axial beabstandet von der Unterlage, auf welcher die Halterung 10 abzustellen ist, zu liegen kommt. Auf diese Art und Weise kann ein mit einer in das Körpergewebe eindringenden Injektionseinrichtung fluidführend in Verbindung stehendes Schlauchstück sozusagen frei hängend an den Auslass 26 der Spritze 18 angekoppelt werden. Hierbei kommen bevorzugt standardisierte Verbindungskomponenten, wie etwa Luer-Lock-Kupplungen zur Anwendung.

In Axialrichtung, welche in sämtlichen Fig. 1 bis 8 im Wesentlichen in Vertikalrichtung verläuft, stützt sich die in der Aufnahme 16 zu liegen kommende Spritze 18 mit ihren radial vom Spritzenkörper 20 hervorstehenden Haltevorsprüngen 28 an einem oberen Endabschnitt 80 des Gehäuses 12 ab. Hierbei ist dieser als Auflager fungierende Endabschnitt 80 etwas unterhalb des Anlageflanschs 30 angeordnet. Dies ist insoweit von Vorteil, als dass auch Anwender mit verhältnismäßig kurzem Daumen einen Kolben 22 einer vergleichsweise großen Spritze, etwa einer 20 ml-Spritze problemlos zu Injektionszwecken betätigen können.

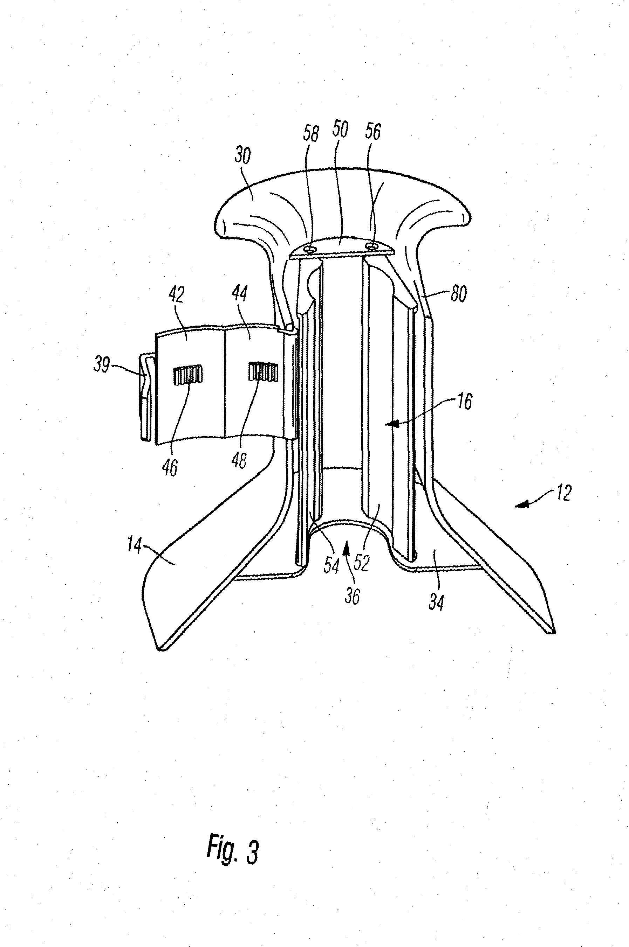

Wie in Fig. 3 ersichtlich, weist das Spritzgussgehäuse 12 im Bereich der konischen Erweiterung des Standfußes 14 eine Stegplatte 34 auf, die einerseits die Verwindungssteifigkeit und Stabilität des Gehäuses 12 erhöht und andererseits als Aufnahme für die Schwenkachsen 56, 58 der beiden Halteflügel 52 und 54 fungieren kann. Die innerhalb der Aufnahme 16 schwenkbar angeordneten Halteflügel 52, 54 sind nach unten an der Stegplatte 34 und nach oben an einem Quersteg 50 angelenkt. Etwaige auf die Halteflügel 52, 54 einwirkende Federelemente 76 sind in den Fig. 1 bis 3 nicht explizit gezeigt. In etwa fluchtend zu der von den Halteflügeln 52, 54 gebildeten Aufnahme 16 ist in der unten liegenden Stegplatte 34 eine Durchgangsöffnung 36 vorgesehen, innerhalb welcher der Spritzenauslass 26 oder je nach Spritzengröße auch der Spritzenkörper 20 zu liegen kommen kann.

In Fig. 3 ist ebenfalls die Innenseite des schwenkbaren Verschlusses 38 gezeigt, welcher an einer Seitenwange des zylindrischen Gehäuseabschnitts um eine Achse 78 schwenkbar angelenkt ist und mit Hilfe eines Rastmittels 39 an der gegenüberliegenden, in Fig. 3 rechts angeordneten Gehäusewange und einem dort vorgesehenen Gegenrastmittel 40 einrasten kann. Der Verschluss 38 ist in zwei Flächensegmente 42, 44 unterteilt, welche jeweils auf ihrer Innenseite eine zumindest sich bereichsweise in Axialrichtung erstreckende und in Umfangsrichtung verlaufende Verzahnung oder Zahnfläche 46, 48 aufweisen.

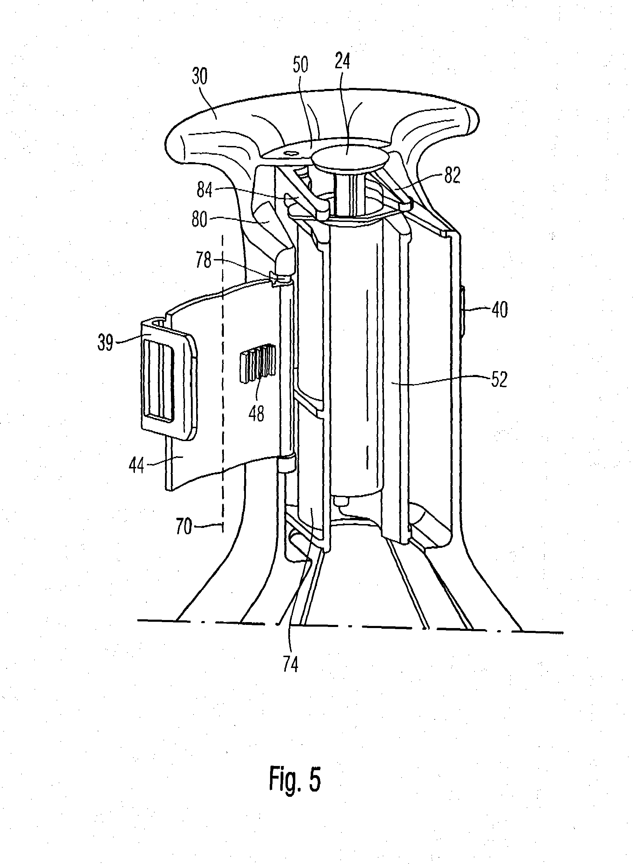

Die beiden Flächensegmente 42, 44 sind dabei biegeelastisch zueinander und schwenkbar ausgebildet. Zwischen den beiden Flächensegmenten verläuft eine, in Fig. 5 gesondert dargestellte Knick- oder Biegeachse 70.

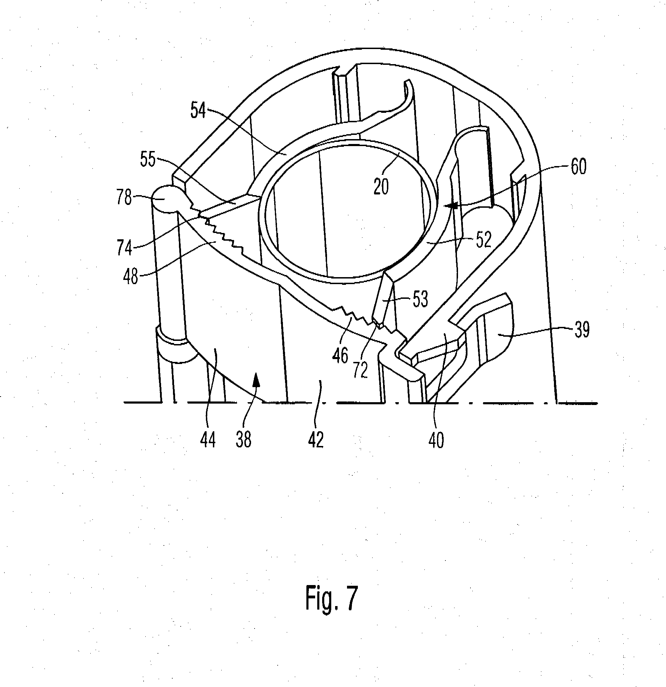

Mit Erreichen seiner in Fig. 7 verdeutlichten Verschlussstellung rastet der Verschluss 38 mit einer Verschlusslasche 39 an einem an der Seitenwange des Gehäuses ausgebildeten Gegenrastelement 40 ein. Es ist hierbei ferner erkennbar, wie die radial nach innen weisenden Zahnflächen 46, 48 mit den korrespondierend hierzu ausgebildeten gefasten Endabschnitten der Halteflügel 52, 54 zusammenwirken. Wird beispielsweise ein radial nach innen gerichteter Druck auf die Flächensegmente 42, 44 des Verschlusses 38 ausgeübt, so bewirkt dies, dass die Halteflügel 52, 54 über die wechselseitige Verzahnung von Zahnfläche 46, 48 und Fase 72, 74 radial nach innen zur Bildung einer Klemmung mit dem Spritzenkörper 20 gedrückt werden. Somit kann die Halterung 10 universell für unterschiedlich große Spritzen und Spritzendurchmesser Verwendung finden.

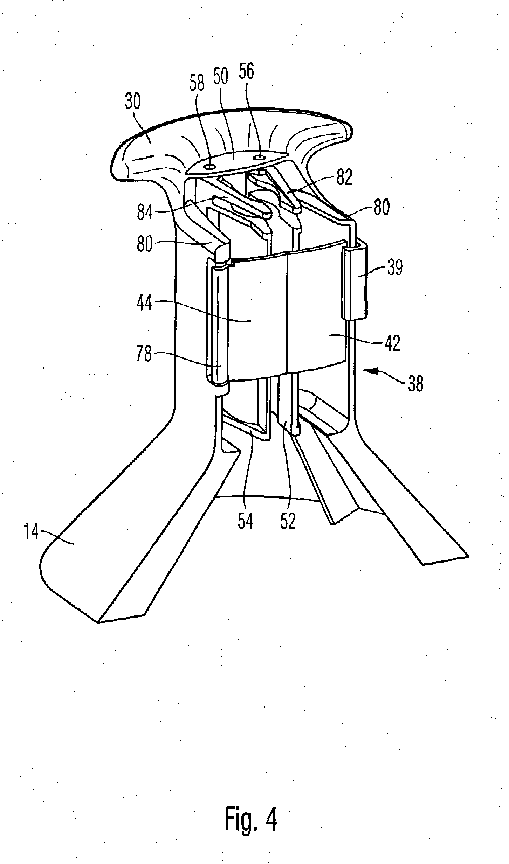

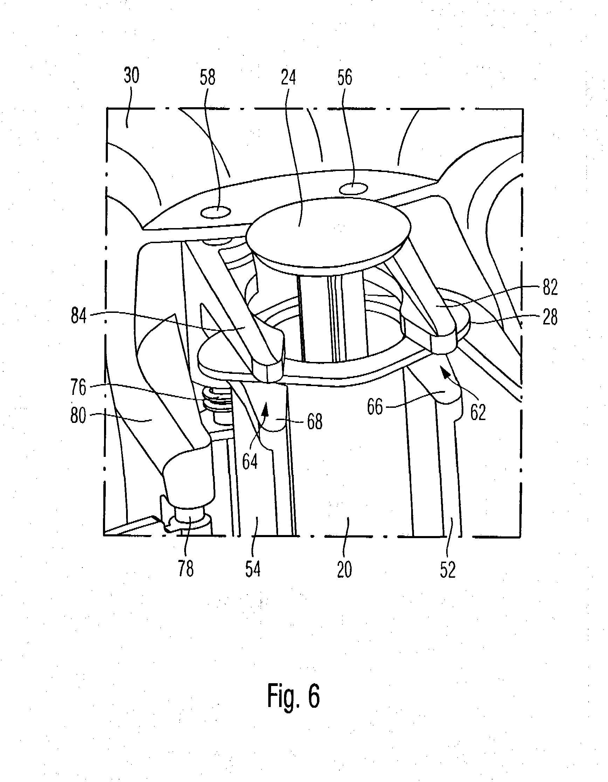

Im Unterschied zur Ausgestaltung gemäß der Fig. 1 bis 3, weist die Ausführungsform nach den Fig. 4 bis 8 im Wesentlichen lediglich andersartig ausgebildete Halteflügel 52, 54 auf, die der Einfachheit halber aber mit gleichen Bezugsziffern versehen sind. Wie in den Fig. 5 und 6 ersichtlich, weisen die Halteflügel 52, 54 in diesem Fall jeweils einen in etwa in Höhe des Auflagers 80 des Gehäuses 12 liegenden Schlitz 62, 64 auf, in welche die diametral gegenüberliegenden Haltevorsprünge 28 des Spritzenkörpers 20 in Radialrichtung eingefügt werden können.

Für ein leichtes Einfügen kann hierbei vorgesehen sein, die Schlitze 62, 64 mit einer Einführschräge 66, 68 zu versehen. Insoweit werden die Halteflügel 52, 54, in Axialrichtung gesehen, zweigeteilt, wobei nunmehr das obere Ende der Halteflügel jeweils von einem fluchtend zum eigentlichen Flügel 52, 54 ausgebildeten Zapfen 82, 84 gebildet wird.

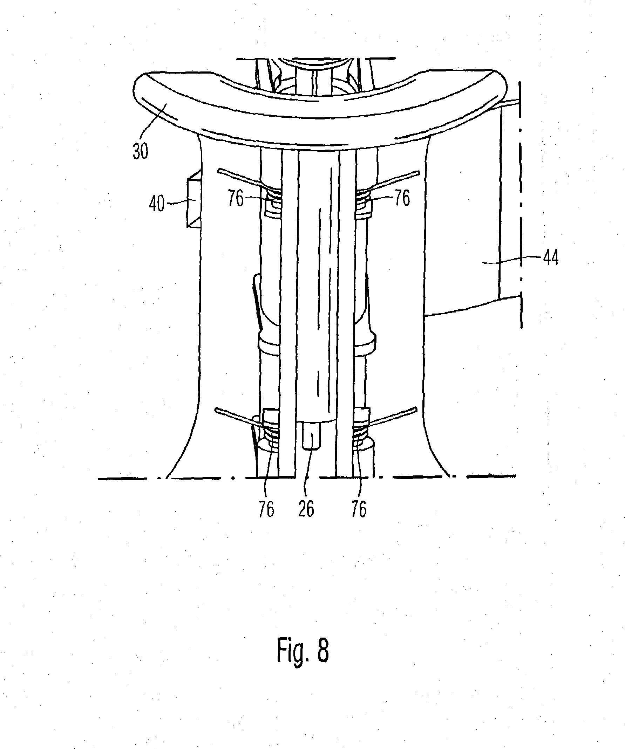

In den Fig. 6 und 8 sind ferner diverse Drehfederelemente 76 gezeigt, mittels derer die Halteflügel 52, 54 unter Federvorspannung stehend am Halter schwenkbar angelenkt sind.

In Fig. 7 ist ferner zu erkennen, dass die Halteflügel 52, 54 bereichsweise konkav ausgebildet sind, um insbesondere mit dem weitgehend zylindrischen Spritzenkörper 20 einen Klemmsitz 60 bilden zu können. Der von den jeweiligen Schwenkachsen 56, 58 beabstandet liegende radiale Endabschnitt der Halteflügel 52, 54 ist dabei vorzugsweise radial nach außen ragend ausgebildet, um die Aufnahme des zylindersymmetrischen Spritzenkörpers 20 zu erleichtern. Die beiden Halteflügel 52, 54 sind beispielsweise vor einem Einsetzen einer Spritze 18 in unmittelbarer Anlagestellung. Die beiden radial nach außen weisenden Enden 53, 55 der jeweiligen Halteflügel 52, 54 bilden eine Art Einführschräge für den Spritzenkörper 20.

Wird die Spritze z.B. in die Aufnahme 16 hineingedrückt, so führt eine solche radial nach innen gerichtete Bewegung des Spritzenkörpers 20 zu einem Aufschwenken der Halteflügel 52, 54. Sobald die Spritze 18 ihre in Fig. 7 gezeigte Endposition erreicht, schwenken die Halteflügel 52, 54 wieder radial nach innen. Durch Verschließen des Verschlusses 38 und der beschriebenen Wechselwirkung der Verzahnungen 46, 48 mit den Endflächen 72, 74 der Halteflügel 52, 54 kann ein ausreichender Klemmsitz 60 zwischen Halteflügeln 52, 54 und Spritzenkörper 20 und ein sichere Fixierung der Spritze in der Halterung 10 bereitgestellt werden.

### Bezugszeichenliste

- 10: Halterung
- 12: Gehäuse
- 14: Standfuß
- 16: Aufnahme
- 18: Spritze
- 20: Spritzenkörper
- 22: Spritzenkolben
- 24: Druckstück
- 26: Auslass
- 28: Haltevorsprung
- 30: Anlageflansch
- 32: Grifffläche
- 34: Steg
- 36: Durchgangsöffnung
- 38: Verschluss
- 39: Rastmittel
- 40: Gegenrastmittel
- 42: Verschlusssegment
- 44: Verschlusssegment
- 46: Zahnfläche
- 48: Zahnfläche
- 50: Quersteg
- 52: Halteflügel
- 53: Endabschnitt
- 54: Halteflügel
- 55: Endabschnitt
- 56: Schwenkachse
- 58: Schwenkachse
- 60: Klemmsitz
- 62: Schlitz
- 64: Schlitz
- 66: Einführschräge
- 68: Einführschräge
- 70: Biegeachse
- 72: Schrägfläche
- 74: Schrägfläche
- 76: Drehfeder
- 78: Schwenkachse
- 80: Auflager
- 82: Zapfen
- 84: Zapfen

## Patentansprüche

1. Halterung für eine Injektionsspritze (18) mit einem zur Aufnahme der Spritze ausgebildeten Gehäuse (12), einem Standfuß (14) zum Abstellen auf einer Unterlage und mit Haltemitteln (38, 52, 54, 62, 64) zum axialen und/oder radialen Fixieren der Spritze (18) an oder in der Halterung, wobei die Haltemittel (38, 52, 54, 62, 64) derart ausgebildet sind, dass ein Auslass (26) einer in der Halterung angeordneten Spritze (18) dem freien Ende des Standfußes (14) zugewandt und beabstandet hiervon zu liegen kommt.

2. Halterung nach Anspruch 1, wobei der Abstand des Auslasses (26) zum Standfußende wenigstens der Axialerstreckung eines SchlauchAnschlussstücks zuzüglich eines Mindest-Schlauchbiegeradius entspricht, bei welchem der mit dem Spritzenauslass in Fluidverbindung tretende Schlauch fluiddurchlässig bleibt.

3. Halterung nach einem der vorhergehenden Ansprüche, wobei das Gehäuse (12) an seinem dem Standfuß (14) abgewandten oberen Endabschnitt einen radial nach außen ragenden, zumindest bereichsweise umlaufenden Anlageflansch (30) aufweist.

4. Halterung nach einem der vorhergehenden Ansprüche, wobei das Gehäuse (12) eine zumindest bereichsweise zylindrische, mit einer außenseitigen Grifffläche (32) versehene Grundstruktur aufweist, die sich zum Standfuß (14) hin radial erweitert.

5. Halterung nach einem der vorhergehenden Ansprüche, wobei das Gehäuse (12) eine radial zugängliche Aufnahme (16) zum Einfügen der Spritze (18) in das Gehäuse (12) aufweist.

6. Halterung nach einem der vorhergehenden Ansprüche, wobei in der Aufnahme (16) zumindest ein Halteflügel (52, 54) schwenkbar gelagert ist, mit welchem die Spritze (18) klemmend in die Halterung bringbar ist.

7. Halterung nach Anspruch 6, wobei der zumindest eine Halteflügel (52, 54) an einer im Wesentlichen parallel zur Axialrichtung verlaufenden Schwenkachse (56, 58) unter Einwirkung zumindest einer Drehfeder (76) gelagert ist.

8. Halterung nach einem der vorherigen Ansprüche 6 oder 7, wobei zwei parallel zueinander verlaufende Halteflügel (52, 54) derart ausgebildet sind, dass die Aufnahme (16) das Einfügen einer Spritze variabler Größe ermöglicht.

9. Halterung nach einem der vorhergehenden Ansprüche 6 bis 8, wobei der zumindest eine Halteflügel (52, 54) in der Ebene senkrecht zur Axialrichtung zumindest bereichsweise eine dem Spritzenkörper (20) angepasste Krümmung aufweist.

10. Halterung nach einem der vorhergehenden Ansprüche 6 bis 9, wobei der zumindest eine Halteflügel (52, 54) an einem seiner Schwenkachse (56, 58) entgegengesetzten Endabschnitt einen im Wesentlichen radial nach außen ragenden und als Einführhilfe für die Spritze (18) fungierenden Endabschnitt (53, 55) aufweist.

11. Halterung nach einem der vorhergehenden Ansprüche 5 bis 10, wobei die für die Spritze (18) vorgesehene Aufnahme (16) mittels eines schwenkbar oder drehbar am Gehäuse (12) gelagerten Verschlussmittels (38) verschließbar ist.

12. Halterung nach Anspruch 11, wobei das Verschlussmittel (38) als ein die Aufnahme (16) in Umfangsrichtung des Gehäuses (12) und in Spritzenlängsrichtung zumindest bereichsweise umschließendes Rastmittel (39) ausgebildet ist, welches mit einem außen am Gehäuse (12) angeordneten Gegenrastmittel (40) zusammenwirkt.

13. Halterung nach einem der vorhergehenden Ansprüche 11 oder 12, wobei das Verschlussmittel (38) an seiner der Spritze (18) zugewandten Innenseite zumindest eine Zahnfläche (46, 48) aufweist, welche dazu ausgebildet ist, mit einer korrespondierend hierzu ausgebildeten endseitigen Schrägfläche (72, 74) des zumindest einen Halteflügels (52, 54) derart zusammen zu wirken, dass der Halteflügel (52, 54) unter Einwirkung einer radial nach innen gerichteten Kraft auf das Verschlussmittel (38) radial nach innen zur Spritze hin verschwenkt.

14. Halterung nach einem der vorhergehenden Ansprüche 11 bis 13, wobei das Verschlussmittel (38) zwei in Umfangsrichtung nebeneinander angeordnete und flexibel miteinander verbundene Flächensegmente (42, 44) aufweist, welche jeweils mit einer innen liegenden Zahnfläche (46, 48) mit einer endseitigen Schrägfläche (72, 74) eines Halteflügels (52, 54) zusammenwirken.

15. Halterung nach Anspruch 11, wobei das Verschlussmittel (38) drehbar um die Axialrichtung im Vergleich zum Gehäuse (12) ausgebildet ist und wobei im Verschlussmittel (38) einen Bereich ausgebildet ist, der nach partieller Drehung um die Spritzenlängsrichtung die Spritze (18) in der Aufnahme (16) des Gehäuses (12) fixiert.

16. Halterung nach einem der vorhergehenden Ansprüche, wobei der zumindest eine Halteflügel (52, 54) und/oder das Gehäuse selbst (12) an ihren oberen, dem Standfuß (14) abgewandten Endabschnitten zumindest einen Schlitz (62, 64) oder ein Auflager (80) für einen radial nach außen ragenden Haltevorsprung (28) einer Spritze (18) aufweisen, um die Spritze (18) zumindest in ihrer Injektionsrichtung axial an der Halterung zu fixieren.
